# DEMANDE DE BREVET EUROPEEN

(11) **EP 4 782 036 A1**
(43) Date de publication de la demande: **29.07.2026**
(21) Numéro de dépôt: 25222577.6
(22) Date de dépôt: 11.12.2025
(51) Int. Cl.: A61M 16/00, A45C 5/00, A61B 50/30

(54) **ENCEINTE DE PROTECTION POUR APPAREIL DE DÉLIVRANCE DE GAZ MÉDICAL, TEL DU NO**

(30) Priorité: 24.01.2025 FR 2500763
(71) Demandeur: INOSYSTEMS, 92160 Antony (FR)
(72) Inventeur: TIRILLY, Nicolas, 92160 Antony (FR)
(74) Mandataire: Air Liquide

(57) **Abrégé**

L'invention concerne une enceinte (1) de protection pour un appareil de délivrance de gaz (100), typiquement un appareil de délivrance de NO, comprenant une enveloppe protectrice (2) comprenant une façade avant (10) et un fond (50) et un compartiment interne (3) dimensionné et configuré pour loger un appareil de délivrance de gaz (100). Une première découpe frontale (6) avec une vitre transparente (7) est aménagée dans la façade avant (10). Un premier élément amortisseur (4) est agencé sur le fond (50), à l'intérieur du compartiment interne (3), et un deuxième élément amortisseur (5) est agencé sur la façade avant (10) et fait saillie en éloignement par rapport à ladite façade avant (10) de l'enveloppe protectrice (2).

## Description

L'invention concerne une enceinte de protection destinée à protéger un appareil de délivrance de gaz médical destiné à une personne, i.e. un patient, typiquement un appareil ou dispositif de délivrance de NO, notamment pendant ses transports et son utilisation.

Dans le cadre de leur traitement médical, certaines personnes, appelées des patients, reçoivent un gaz thérapeutique qui leur est administré au moyen d'un appareil ou d'une installation d'administration de gaz alimentant un circuit patient, fournissant le gaz thérapeutique à une interface respiratoire, tel un masque respiratoire, une sonde d'intubation trachéale ou autre, servant à administrer le gaz thérapeutique aux voies aériennes des patients.

Il en est ainsi du monoxyde d'azote gazeux inhalé ou « NO », en particulier d'un mélange gazeux combiné NO/N₂/O₂ obtenu par injection d'un mélange gazeux NO/N₂ contenant typiquement entre 100 et 2000 ppmv de NO (reste N₂) dans un flux d'air ou de mélange O₂/N₂ contenant au moins 20%vol. d'O₂ provenant d'un ventilateur médical et véhiculé par le circuit patient, tel un tuyau flexible ou analogue.

Le mélange combiné NO/N₂/O₂, en particulier le NO qu'il contient (e.g. dose de < 80 ppmv), permet de dilater les vaisseaux pulmonaires et d'augmenter l'oxygénation en améliorant les échanges gazeux chez le patient.

Il est dès lors utilisé pour traiter l'Hypertension Artérielle Pulmonaire du nouveau-né ou PPHN (pour *Persistent Pulmonary Hypertension of the Newborn*)*,* le Syndrome de Détresse Respiratoire Aigüe ou SDRA chez l'adulte ou encore les hypertensions pulmonaires en chirurgie cardiaque, comme enseigné notamment par EP-A-560928, EP-A-1516639 ou US-A-10,201,564.

Pour injecter le mélange gazeux NO/N₂ dans le circuit patient et réaliser le mélange gazeux combiné NO/N₂/O₂, on utilise typiquement un appareil de délivrance de NO, comme décrit par exemple par EP3906955, EP4209243 ou EP4241812.

Comme rappelé par EP4410345, certains appareils de fourniture, i.e. délivrance, de gaz, telle NO, doivent pouvoir être transportés et utilisés sur site d'intervention par les équipes de secours (SAMU, pompiers...) et/ou dans des véhicules d'urgence, tel qu'une ambulance, un avion, un hélicoptère ou autre.

Or, lors de son transport, de ses manipulations ou analogues, un tel appareil de fourniture de gaz est souvent soumis à des chocs, des salissures, des chutes ou autres conditions météorologiques ou environnementales difficiles (e.g. pluie, neige, vent, boue...) susceptibles de le salir, de l'endommager et/ou de le détériorer jusqu'à le rendre inutilisable, ce qui engendre un problème évident de sécurité pour le patient à qui le gaz thérapeutique ne peut plus alors être distribué.

D'autres sacoches, enceintes ou analogues de rangement ou de transport de dispositifs ou appareils médicaux sont enseignés par CN221654587, DE202010016037, DE202011005093, EP3791911 et DE102008064480.

Un problème est de pouvoir éviter ou diminuer le risque de salissure et/ou détérioration d'un appareil de fourniture de gaz, typiquement un appareil de délivrance de NO, en particulier lorsqu'il est utilisé sur le terrain, i.e. sur site d'intervention (i.e. hors hôpital) par une équipe de secours (SAMU, pompiers...) et/ou dans un véhicule d'urgence (par exemple ambulance, avion, hélicoptère ...).

De plus, l'appareil de fourniture de gaz doit pouvoir continuer à être utilisé normalement, malgré ces conditions d'utilisation difficiles, en particulier l'utilisateur doit pouvoir accéder normalement à l'écran d'affichage de l'appareil, aux boutons de réglage ou de sélection éventuels, aux connecteurs pneumatiques et/ou électriques servant aux différents raccordements ou branchements...

Une solution de l'invention concerne une enceinte de protection pour un appareil de délivrance de gaz médical à une personne, i.e. un patient, typiquement un appareil de délivrance de NO, comprenant une enveloppe protectrice comprenant une façade avant et un fond, et un compartiment interne dimensionné et configuré pour loger un appareil de délivrance de gaz, typiquement un appareil de délivrance de NO.

De plus, l'enceinte de protection, en particulier l'enveloppe protectrice, comprend :
- une première découpe frontale, aussi appelée fenêtre frontale, aménagée dans la façade avant de l'enveloppe protectrice, et une vitre transparente en polymère agencée dans ladite première découpe,
- un premier élément amortisseur est agencé sur le fond, à l'intérieur du compartiment interne, et
- un deuxième élément amortisseur est agencé sur la façade avant et fait saillie en éloignement par rapport à ladite façade avant de l'enveloppe protectrice, c'est-à-dire qu'il fait saillie vers l'extérieur.

Selon le mode de réalisation considéré, l'enceinte de protection de l'invention peut comprendre l'une ou plusieurs des caractéristiques suivantes :
- la vitre transparente est souple, c'est-à-dire flexible.
- l'enveloppe protectrice forme une coque ou structure périphérique protectrice au sein de laquelle est agencé, i.e. logée, l'appareil.
- lorsqu'un appareil est logé dans l'enveloppe protectrice, il repose sur le premier élément amortisseur agencé sur le fond.
- l'enveloppe protectrice est formée d'une toile comprenant des fibres textiles synthétiques, de préférence des fibres en polyester.
- de préférence les fibres textiles synthétiques sont enduites d'une substance protectrice (i.e. toile enduite), de préférence un polymère, par exemple du polychlorure de vinyle ou PVC.
- l'enveloppe protectrice comprend une façade avant, une face arrière, un côté droit, un côté gauche et un fond, reliés les uns aux autres, c'est-à-dire solidaires les uns des autres.
- l'enveloppe protectrice comprend ou est formée d'une toile associée à, i.e. renforcée par, au moins une plaque de renfort, par exemple en polymère, typiquement une plaque rigide.
- la plaque protectrice est agencée dans la face arrière.
- l'enveloppe protectrice comprend en outre un toit en fibres textile synthétique qui protège la partie haute du produit, tout en laissant l'accès à la poignée de transport.
- l'appareil comprend une poignée de transport et le toit de l'enveloppe protectrice vient se positionner sous la poignée, c'est-à-dire entre la poignée et la coque de l'appareil surmontée de ladite poignée de transport.
- le toit assure une protection étanche de la partie supérieure de l'appareil.
- l'enveloppe protectrice a une largeur (L) comprise entre 35 et 40 cm, typiquement entre 28 cm et 33 cm environ, une profondeur (P) comprise entre 8 et 20 cm, typiquement entre 10 cm et 18 cm environ, et une hauteur (H) comprise entre 18 et 40 cm, typiquement entre 20 cm et 34 cm environ.
- la façade avant, la face arrière, le côté droit, le côté gauche et le fond, et préférentiellement le toit, comprennent des parois, typiquement une paroi avant, une paroi arrière, une paroi de côté droit, une paroi de côté gauche et une paroi de fond, et préférentiellement une paroi de toit.
- le compartiment interne est délimité par lesdites parois.
- la façade avant, la face arrière, le côté droit, le côté gauche sont solidaires du fond et se projettent vers le haut au-dessus du fond.
- la façade avant et la face arrière sont reliées l'une à l'autre par le côté droit et le côté gauche.
- la façade avant et la face arrière sont agencées parallèles ou sensiblement parallèles l'une à l'autre c'est-à-dire qu'elles se font face en étant parallèles.
- selon un autre mode de réalisation, la façade avant et la face arrière sont agencées de sorte que l'une est légèrement inclinée par rapport à l'autre c'est-à-dire qu'elles se font face sans être parallèles.
- le côté droit et le côté gauche sont agencés de manière à se faire face l'un à l'autre, i.e. parallèles ou non l'un à l'autre.
- la paroi de toit ferme le compartiment interne par le haut.
- la paroi de toit est souple, c'est-à-dire flexible.
- la paroi de toit (ou toit) est pivotante par rapport à la façade avant, de préférence au niveau d'une région d'angle formant charnière, typiquement située en haut de la façade avant.
- le premier élément amortisseur recouvre la majeure partie de la surface intérieure du fond, de préférence toute la surface intérieure du fond.
- le premier élément amortisseur a une forme de trapèze ou de parallélépipède rectangle, de préférence de trapèze.
- le fond a une forme de de trapèze ou de parallélépipède rectangle, de préférence de trapèze.
- le premier élément amortisseur a une longueur comprise entre 10 cm et 15 cm, une largeur comprise entre 22 cm et 31 cm et une épaisseur comprise inférieure à 5 cm, de préférence de l'ordre de 2 cm à 3 cm environ.
- le deuxième élément amortisseur a une hauteur comprise entre 5 cm et 10 cm, une largeur comprise entre 10 cm et 15 cm et une épaisseur/profondeur comprise entre 1 cm et 3 cm.
- un troisième élément amortisseur est agencé sur la façade avant et fait saillie en éloignement par rapport à ladite façade avant de l'enveloppe protectrice.
- le troisième élément amortisseur a une hauteur comprise entre 5 cm et 10 cm, une largeur comprise entre 1 cm et 3 cm et une épaisseur/profondeur comprise entre 1 cm et 3 cm.
- selon un mode de réalisation, le deuxième élément amortisseur et le troisième élément amortisseur ont une hauteur (approximativement) égale.
- les deuxième et troisième éléments amortisseurs sont positionnés sur la façade avant, entre le fond et la première découpe frontale comprenant la vitre transparente.
- une deuxième découpe frontale est aménagée dans la façade avant de l'enveloppe protectrice.
- la deuxième découpe frontale est située entre les deuxième et troisième éléments amortisseurs.
- le premier, deuxième et/ou le troisième élément(s) amortisseur(s) comprennent une mousse amortissante, par exemple en caoutchouc cellulaire, de préférence en caoutchouc nitrile (NBR).
- la paroi de toit est par exemple formée d'un tissu en fibres textile synthétiques.
- la paroi de toit est configurée pour pivoter entre (au moins) une position dite de « fermeture », dans laquelle l'utilisateur n'a pas accès à l'intérieur du compartiment, et une position dite « d'ouverture », dans laquelle l'utilisateur peut avoir accès à l'intérieur du compartiment interne.
- un système de fermeture assure un maintien de la paroi de toit en position de « fermeture ».
- le système de fermeture comprend un ou des connecteurs ou analogues, de préférence deux connecteurs.
- chaque connecteur comprend une partie femelle et une partie mâle venant s'insérer l'une dans l'autre pour former un système de fermeture de type mâle/femelle.
- la partie mâle de chaque connecteur est portée par la face ou paroi arrière de l'enveloppe protectrice et la partie femelle de chaque connecteur est portée par la paroi de toit, ou inversement.
- le ou les connecteurs, en particulier les parties mâles et femelles de chaque connecteur(s), sont agencés sur une ou des sangles.
- elle comprend deux connecteurs agencés sur deux sangles agencées parallèles l'une à l'autre et espacées d'une distance d'environ 15 cm à 25 cm.
- la partie mâle de chaque connecteur est solidarisée ou portée par une sangle (ou une partie de sangle) solidaire de la paroi de la paroi arrière de l'enveloppe protectrice, i.e. de l'enceinte.
- les deux sangles portant les parties mâles des deux connecteurs coopèrent avec deux zones d'attache situées sur la surface de la paroi arrière, typiquement des zones allongées, de manière à y être retenues par fixation via un système d'attache ajustable à deux bandes auto-agrippantes présentant des textures différentes l'une de l'autre mais complémentaires de sorte d'obtenir une liaison ou attache amovible lorsqu'elles sont mises en contact l'une avec l'autre, typiquement un système de type attache Velcro^{®}.
- les deux sangles portant les parties femelles des deux connecteurs sont solidaires, i.e. fixées, au toit de l'enveloppe protectrice, i.e. de l'enceinte.
- la face arrière de l'enveloppe protectrice comprend une large ouverture centrale ou fenêtre arrière donnant accès au compartiment interne, de préférence la fenêtre arrière est située entre les deux sangles.
- le côté droit de l'enveloppe protectrice comprend plusieurs petits panneaux juxtaposés les uns aux autres, aussi appelés « carreaux » ou « tuiles », en particulier agencés de sorte de se superposer partiellement et ainsi permettre d'évacuer l'eau de ruissellement et d'éviter que celle-ci ne pénètre dans l'enveloppe.
- les petits panneaux viennent se rattacher à l'enceinte, via leurs bords droits et gauches, en particulier au niveau des bordures verticales du côté droit de l'enceinte.
- au moins une partie des bords supérieurs et/ou inférieurs des petits panneaux sont libres, i.e. non fixés, de manière à former des fentes ou des « ouïes » donnant accès à l'intérieur du compartiment interne.
- une partie des petits panneaux est formée d'un matériau polymère transparent souple, c'est-à-dire qu'ils sont vitrés et transparents.
- une partie des petits panneaux est formée d'un matériau souple opaque, tel un tissu en fibres textile synthétiques.
- le côté gauche de l'enveloppe protectrice comprend une découpe latérale où est agencée une vitre transparente, de préférence souple, de sorte de former une fenêtre vitrée latérale gauche, permettant à l'utilisateur de voir l'intérieur de l'enceinte.
- la vitre latérale transparente s'étend sur la majeure partie de la surface du côté gauche.
- la ou les vitres transparentes, de préférence souples, sont formé d'une feuille, plaque, toile, bâche ou analogue polymère transparent, de préférence en PVC, par exemple un PVC type vitral.
- la vitre latérale transparente comprend un ou des petites ouvertures aménagées au travers de ladite vitre latérale transparente, par exemple de forme circulaire, ovale ou autre.
- tout ou partie des quatre faces ou parois périphériques de l'enveloppe est percée ou comprend, typiquement à quelques mm au-dessus du fond (par exemple à environ 2 cm du fond), une rangée de trous ou orifices en communication fluidique avec le compartiment interne, pour permettre une évacuation de l'eau éventuelle pouvant se retrouver dans ledit compartiment interne. A l'inverse, si les trous sont trop bas, c'est-à-dire trop proche du fond, et que l'on pose le produit dans une flaque d'eau par exemple, de l'eau va pénétrer dans le produit, ce qu'il faut éviter car l'eau peut détérorer le ventilateur se trouvant à l'intérieur de l'enveloppe. Toutefois, on peut accepter qu'il y ait un peu d'eau au fond tant qu'elle est suffisamment éloignée du ventilateur à protéger.
- les deuxième et troisième éléments amortisseurs positionnés sur la façade avant, sont recouverts de tissu, en particulier du même tissu que celui formant la face avant de l'enveloppe.

Par ailleurs, l'invention porte aussi sur un ensemble de ventilation comprenant un appareil de délivrance de gaz, typiquement un appareil de délivrance de NO, logé dans l'enveloppe protectrice d'une enceinte de protection selon l'invention, en particulier celle décrite ci-avant.

Autrement dit, l'invention concerne également une utilisation d'une enceinte de protection selon l'invention, pour protéger un appareil de délivrance de gaz, typiquement un appareil de délivrance de NO, en particulier celle décrite ci-avant.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une enceinte de protection pour appareil de délivrance de gaz, ici de NO, selon l'invention, en vue de 3/4, côté droit.
Fig. 2 est une vue de 3/4, côté gauche de l'enceinte de protection de Fig. 1.
Fig. 3 est une vue de la façade avant de l'enceinte de protection de Fig. 1.
Fig. 4 est une vue du côté gauche de l'enceinte de protection de Fig. 1.
Fig. 5 est une vue du côté droit de l'enceinte de protection de Fig. 1.
Fig. 6 est une vue de la face arrière de l'enceinte de protection de Fig. 1.
Fig. 7 schématise un appareil de délivrance de NO pouvant être protégé par l'enceinte de protection de Fig. 1.

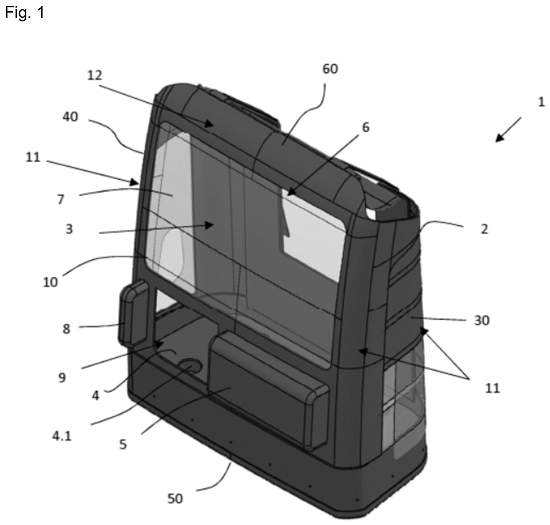

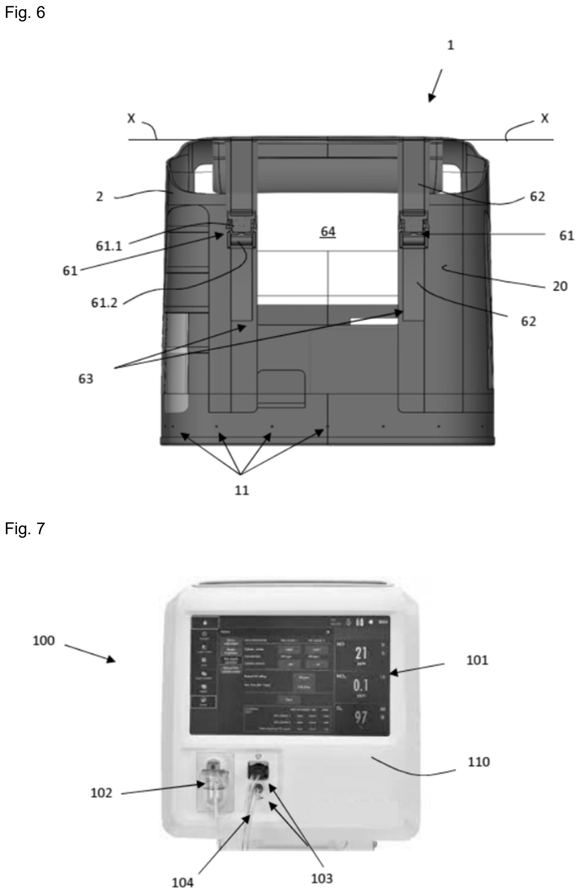

Fig. 1 à Fig. 6 proposent différentes vues d'un mode de réalisation d'une enceinte de protection 1 selon l'invention destinée à loger appareil de délivrance de gaz, à savoir ici un appareil de délivrance de NO 100, tel que montré en Fig. 7.

L'enceinte de protection 1 selon l'invention comprend une enveloppe protectrice 2 comprenant différentes parois 10-60 délimitant un compartiment interne 3 dimensionné et configuré pour loger l'appareil de délivrance de gaz 100 de Fig. 7, i.e. de NO. Ici, il s'agit de l'appareil de délivrance de NO 100 commercialisé sous l'appellation SoKinox^{®}.

Plus précisément, l'enveloppe protectrice 2 comprend :
- une face ou façade avant 10 et une face arrière 20, agencées face à face et séparées l'une de l'autre par le compartiment interne 3,
- un côté droit 30 et un côté gauche 40, aussi agencés face à face. Le côté droit 30 et le côté gauche 40 joignent chacun la façade avant 10 et la face arrière 20, au niveau de zones ou régions d'angle 11, en étant séparés l'un de l'autre par le compartiment interne 3.
- un fond 50 surmonté par les parois formant la façade avant 10, la face arrière 20, et les côtés droit 30 et gauche 40, lesquels parois se projettent vers le haut à partir du fond 50.
- une paroi supérieure ou paroi de toit 60 fermant le compartiment interne 3 par le haut. La paroi de toit 50 est montée pivotante par rapport à la façade avant 10, au niveau d'une région d'angle formant charnière 12 (axe XX) située en haut de la façade avant 10.

La paroi de toit 60 est préférentiellement souple pour permettre son pivotement atour de l'axe XX de la charnière 12 entre (au moins) :
- une position dite de « fermeture », illustrée en Fig. 6, dans laquelle l'utilisateur n'a pas accès à l'intérieur du compartiment 3, et
- une position dite « d'ouverture », dans laquelle l'utilisateur peut avoir accès à l'intérieur du compartiment 3 pour y insérer ou en extraire l'appareil de délivrance de NO 100.

Bien entendu, lors de son pivotement entre ces deux positions, la paroi de toit 60 peut adopter d'autres positions angulaires intermédiaires.

La paroi de toit 60 peut être maintenue en position de « fermeture » par un (des) système de fermeture adapté, par exemple deux connecteurs 61 ou analogues comprenant chacun une partie femelle 61.1 portée par la paroi de toit 60 dans laquelle vient s'insérer dans une partie mâle 61.2 portée par la face ou paroi arrière 20, ou inversement, comme illustré en Fig. 6. Les connecteurs 61 peuvent être portés par des sangles 62 ou analogue, elles-mêmes solidaires de la paroi de toit 60 et de la paroi arrière 20.

L'enveloppe protectrice 2 est formée par exemple d'un tissu en fibres textile synthétique, de préférence en polyester ou polypropylène, pouvant être enduit d'une protection, de préférence du PVC, et pouvant être associé à une plaque de renfort en plastique rigide, de préférence en polypropylène ou en polycarbonate.

La plaque de renfort est située dans la face arrière, c'est-à-dire forme tout ou partie de ladite face arrière.

Elle peut être formée de plusieurs parois ou sous-unités, i.e. des panneaux ou des éléments de panneaux, assemblés les uns aux autres, par collage, thermo-soudage, couture ou autre.

Ici, l'enveloppe protectrice 2 est formée de quatre panneaux ou parois périphériques formant la façade avant 10, la face arrière 20 et les côtés droit et gauche, lesquels sont portés par le fond 50 et solidaires de celui-ci.

Comme illustré sur Fig. 1 à Fig. 3, la façade avant 10 comprend une première découpe frontale 6, c'est-à-dire une ouverture avant, traversant sa paroi, dans laquelle est agencée une vitre transparente 7, i.e. une vitre frontale, typiquement en polymère, de faible épaisseur, typiquement < 1 mm, de préférence une vitre transparente 7 souple, c'est-à-dire flexible.

La découpe avant 10 et la vitre transparente 7 forment une fenêtre vitrée, i.e. une fenêtre avant, permettant à l'utilisateur de voir l'intérieur de l'enceinte 1.

Par exemple, la première découpe frontale 6 peut avoir une largeur de 24 cm à 26 cm et une hauteur de 14 cm à 16 cm. La vitre transparente 7 recouvre toute la surface de la première découpe frontale 6 et/ou à sensiblement les mêmes dimensions.

Cette découpe frontale 6 formant une ouverture avant, est destinée à venir se positionner face à l'écran d'affichage 101 de l'appareil de délivrance de NO 100, lorsqu'il est logé dans le compartiment interne 3, de manière à permettre à l'utilisateur de visualiser les affichages (i.e. informations, données, alarmes, courbes, valeurs, doses....) s'opérant sur l'écran d'affichage de l'appareil 100, pendant son utilisation pour traiter un patient, et/ou d'opérer des choix, des sélections ou autres, via des appuis digitaux de la part de l'utilisateur opérés sur l'écran d'affichage 101 de l'appareil 100, au travers de la vitre transparente 7, lorsque l'écran d'affichage 101 est un écran à dalle tactile.

Le compartiment interne 3 comprend un premier élément amortisseur 4 positionné sur le fond 50, à l'intérieur du compartiment interne 3, sur lequel vient reposer l'appareil de délivrance de NO 100, lorsqu'il est logé dans le compartiment interne 3. Ce premier élément amortisseur 4 peut être amovible.

Le premier élément amortisseur 4 épouse les contours internes du compartiment 3 de l'enveloppe protectrice 2 et la surface interne du fond 50. Il peut être par exemple formé d'une plaque ou analogue s'étendant sur toute ou partie du fond 50 du compartiment interne 3, de préférence sur tout le fond 50, du côté intérieur.

Ce premier élément amortisseur 4 peut être en un matériau amortisseur comme une mousse amortissante, par exemple en caoutchouc cellulaire type NBR ou analogue permettant d'absorber les chocs, les vibrations ou autres, en particulier d'amortir les chutes éventuelles sur le sol, en particulier les chocs au niveau du fond 50, typiquement une chute sur le fond 50 d'une hauteur jusqu'à environ 50 cm.

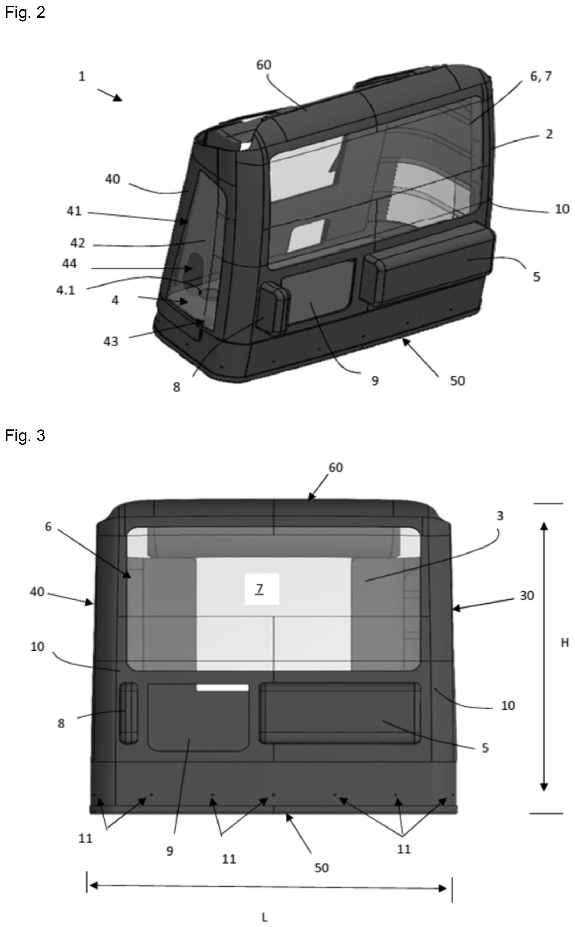

Avantageusement, le premier élément amortisseur 4 peut comprendre des petits logements ou évidements 4.1 destinés à recevoir les pieds de l'appareil 100 lorsqu'il y est positionné, par exemple quatre évidements 4.1 pratiqué dans la surface supérieure du premier élément amortisseur, i.e. dans la surface de la mousse ou analogue, pour recevoir les quatre pieds de l'appareil 100, comme visible en Fig. 1 et Fig. 2.

Par ailleurs, la façade avant 10 comprend en outre un deuxième élément amortisseur 5 agencé sur la façade avant 10, lequel fait saillie en éloignement par rapport à ladite façade avant 10 de l'enveloppe protectrice 2, c'est-à-dire se projetant vers l'extérieur.

Avantageusement, la façade avant 10 peut comprendre un troisième élément amortisseur 8, aussi agencé sur la façade avant 10 et faisant saillie en éloignement par rapport à ladite façade avant 10 de l'enveloppe protectrice 2, c'est-à-dire se projetant lui aussi vers l'extérieur.

Les deuxième et troisième éléments amortisseurs 5, 8 sont eux aussi destinés à absorber ou amortir les chocs, les vibrations ou autres, notamment en cas d'une chute éventuelle sur le sol susceptible de détériorer la face avant 110 et l'écran 101 de l'appareil de délivrance de NO qui se trouve dans le compartiment 3.

Les deuxième et troisième éléments amortisseurs 5, 8 peuvent être formés du même matériau amortisseur que le premier élément amortisseur 4 ou d'un autre matériau telle une mousse de polyéthylène (PE), une mousse en éthylène-acétate de vinyle (EVA) ou analogue.

Le deuxième élément amortisseur 5 a une taille supérieure à celle du troisième élément amortisseur 8, par exemple, le deuxième élément amortisseur 5 a une largeur comprise entre 1 et 3 cm, une hauteur comprise entre 6 et 7 cm et une profondeur comprise entre 2 cm et 3 cm, alors que le troisième élément amortisseur 8 a une largeur comprise entre 12 et 14 cm environ, une hauteur comprise entre 6 et 7 cm environ, et une profondeur comprise entre 2 et 3 cm environ.

Comme on le voit sur Fig. 3, les deuxième et troisième éléments amortisseurs 5, 8 sont situés sous la fenêtre vitrée 6, 7, c'est-à-dire entre la fenêtre vitrée 6, 7 et le fond 50 de l'enveloppe protectrice 2 de l'enceinte 1.

Par ailleurs, une deuxième découpe frontale 9 est aménagée dans la paroi de façade avant 10 de l'enveloppe protectrice 2 comme visible sur Fig. 1 à Fig. 3. Elle se situe entre les deuxième et troisième éléments amortisseurs 5, 8 et donc aussi la fenêtre vitrée 6, 7.

Cette deuxième découpe frontale 9 permet d'avoir accès à l'intérieur du compartiment interne 3, c'est-à-dire à l'appareil situé dans le compartiment interne 3 de l'enveloppe protectrice 2, en particulier à des éléments situés sous l'écran 101 de l'appareil 100 comme un dispositif de type trappe à eau 103, des ports de raccordement 103 de tuyaux ou de câbles électriques 104, ou autres, comme illustré en Fig. 7.

Les deuxième et troisième éléments amortisseurs 5, 8 permettent aussi de protéger le dispositif de type trappe à eau 103 et les ports de raccordement 103 de l'appareil 100, en cas de chute sur sa face avant 10.

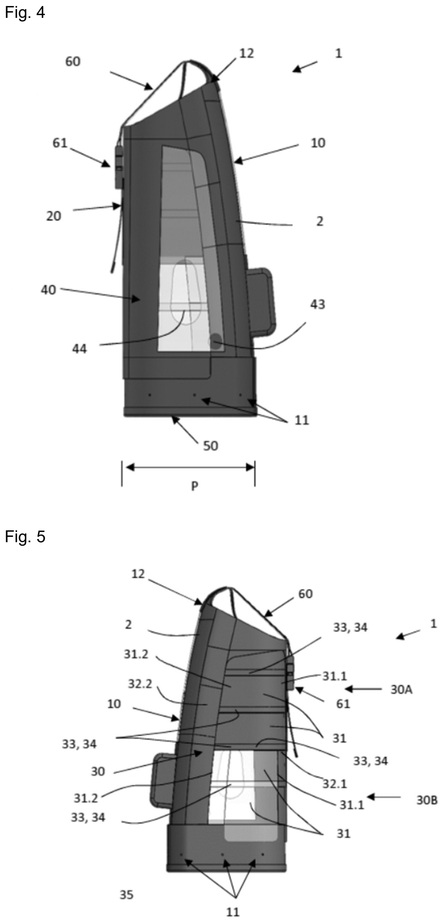

Fig. 1 et Fig. 5 montrent le côté droit 30, i.e. paroi latérale droite, de l'enveloppe protectrice 2 de l'enceinte de protection 1 de l'invention.

Comme on le voit, le côté droit 30 comprend plusieurs petits panneaux 31 juxtaposés les uns aux autres, aussi appelés « carreaux », en particulier agencés ici les uns au-dessus des autres.

Les petits panneaux 31, i.e. leurs bords droits 31.1 et gauches 31.2, viennent se rattacher à l'enceinte 2 au niveau des bordures verticales 32.1, 32.2, respectivement, du côté droit 30 de l'enceinte 2, comme illustré en Fig. 5.

Par contre, au moins une partie des bords 33 supérieurs et inférieurs des petits panneaux 31 ne sont pas fixés les uns autres de manière à former des fentes 34 ou des « ouïes » donnant accès à l'intérieur du compartiment interne 3. Ces fentes 34 permettent le passage de câbles ou de tuyaux.

Certains petits panneaux 31 peuvent se recouvrir légèrement, i.e. se superposer partiellement, dans les régions de leurs bords 33 supérieurs et inférieurs, de manière à améliorer la protection contre les intrusions de polluants ou les intempéries, en particulier contre la pluie, la neige ou analogue.

Les petits panneaux 31 peuvent avoir différentes formes, notamment des formes trapézoïdales, carrées, rectangulaire ou autre. Par exemple, ils peuvent avoir une largeur comprise entre 7 et 10 cm environ, et une hauteur comprise entre 2 et 5 cm environ.

Comme on le voit en Fig. 5, les petits panneaux 31 situés dans la partie haute 30A du côté droit 30, par exemple 2 ou 3 panneaux 31, peuvent être formés d'un matériau tissé et opaque, par exemple une toile en polyester et élastodiène, préférentiellement élastique, typiquement une bande élastique.

Par ailleurs, les petits panneaux 31 situés dans la partie basse 30B du côté droit 30, par exemple 2 ou 3 panneaux 31, sont préférentiellement formés d'un matériau polymère transparent, de préférence souple, formant une petite vitre 35 analogue à la vitre transparente 7, c'est-à-dire la vitre frontale.

Ces petits panneaux 31 vitrés 35 permettent à l'utilisateur de lire des informations présentes sur l'appareil 100 lorsqu'il est agencé dans le compartiment interne 3, comme une étiquette produit ou autre. Là encore, la fente 34 située entre deux petits panneaux 31 vitrés 35 donne accès à l'intérieur du compartiment interne 3 et permet le passage de câble ou de tuyau.

Fig. 2 et Fig. 4 montrent le côté gauche 40, i.e. paroi latérale gauche, de l'enveloppe protectrice 2 de l'enceinte de protection 1 de l'invention.

Comme on le voit, le côté gauche 40 comprend, de manière analogue à la façade avant 10, une découpe latérale 41 traversant sa paroi, dans laquelle est aussi agencée une vitre transparente 42, de préférence souple, i.e. une vitre latérale, typiquement en polymère, de faible épaisseur, typiquement < 1mm. La découpe latérale 41 et la vitre transparente 42 forment, là encore, une fenêtre vitrée, i.e. une fenêtre latérale gauche, permettant à l'utilisateur de voir l'intérieur de l'enceinte 1.

Cette vitre latérale transparente 42 s'étend sur la majeure partie de la surface du côté gauche 40. Elle a ici une forme sensiblement trapézoïdale mais pourrait avoir une autre forme, par exemple rectangulaire ou autre.

Toutefois, il est à noter qu'ici, deux petites ouvertures 43, 44, à savoir ici une ouverture de forme circulaire 43 et une ouverture oblongue 44 en forme de « poire », sont aménagées au travers de la vitre latérale transparente 42, lesquels permettent de faire passer un ou des câbles ou encore de loger un bouton rotatif de réglage par exemple.

En outre, Fig. 6 montre la face arrière 20 de l'enveloppe protectrice 2 de l'enceinte de protection 1 selon l'invention.

La face arrière 20 comprend, comme déjà expliqué, les connecteurs 61 servant au maintien de la paroi de toit 60 en position de fermeture.

Préférentiellement, la partie mâle 61.2 des deux connecteurs 61 sont solidarisés à des sangles 62 ou analogue, elles-mêmes solidaires de la paroi de la paroi arrière 20, comme illustré en Fig. 6. Les deux sangles 62 sont agencées parallèles l'une à l'autre et espacées d'une distance d'environ 15 à 25 cm environ.

Préférentiellement, les deux sangles 62 coopèrent avec deux zones d'attache 63 situées sur la surface de la paroi arrière 20, typiquement des zones allongées, i.e. orientée verticalement, de manière à y être retenues par fixation via un système d'attache ajustable à deux bandes auto-agrippantes présentant des textures différentes l'une de l'autre mais complémentaires de sorte d'obtenir une liaison ou attache amovible lorsqu'elles sont mises en contact l'une avec l'autre, typiquement un système d'attache à petits crochets portés par l'une des bandes et à petites boucles portées par l'autre des bandes, lesquels petits crochets et petites boucles coopèrent ensemble pour assurer le maintien, i.e. un système de type attache Velcro^{®}.

La face arrière 20 est préférentiellement plus rigide que la façade avant 10 et les panneaux latéraux 30, 40 et de toit 60. La face arrière 20 peut être formée d'un matériau plus rigide, comme une plaque de renfort en plastique rigide, de préférence en polypropylène ou en polycarbonate et/ou comprendre des raidisseurs comme des surépaisseurs de matières ou de mousse.

Par ailleurs, on voit que la face arrière 20 comprend une large ouverture centrale ou fenêtre arrière 64, située entre les deux sangles 62, qui donne accès au dos de l'appareil 100, lorsqu'il est logé dans le compartiment interne 3.

En effet, un appareil de délivrance de NO 100 comprend classiquement une platine arrière appelée platine Vesa^{®}, servant au montage de l'appareil 100 sur un support.

La fenêtre arrière 64 est donc configurée et dimensionnée pour permettre d'utiliser la platine Vesa^{®} sans avoir à enlever l'enceinte 1 protégeant l'appareil 100, c'est-à-dire de pouvoir monter l'appareil 100 sur un support tout en continuant à le protéger grâce à l'enceinte protectrice 1.

Dès lors, la fenêtre arrière 64 est préférentiellement de forme rectangulaire, carrée ou analogue. Ses dimensions sont d'environ 140 cm x 150 cm.

Par ailleurs, Fig. 1 à Fig. 6 montrent que les 4 côtés 10-40 de l'enveloppe 2 formant sa paroi périphérique sont percés ou comprennent à leur base, c'est-à-dire juste au-dessus du fond 50, d'une rangée de trous ou orifices 11 permettant une évacuation de l'eau éventuelle qui pourrait entrer ou se retrouver dans le compartiment interne 3 de l'enveloppe 2, lors d'une utilisation ou intervention à l'extérieur par mauvaise météo, par exemple en cas de pluie ou de neige.

Plus généralement, l'enceinte 1 de protection peut avoir une largeur L comprise entre 23 et 33 cm, une profondeur P comprise entre 10 et 19 cm, et une hauteur H comprise entre 27 et 34 cm, afin de pouvoir accueillir un appareil de délivrance de gaz, typiquement un appareil de délivrance NO 100.

De plus, l'enceinte 1 de protection est préférentiellement légère pour permettre son transport aisé, par exemple elle peut avoir un poids (masse) inférieur à 2 kg, de préférence inférieur à 1,5 kg, typiquement compris entre environ 0.7 kg et 1 kg.

L'enceinte 1 de protection présente les avantages de :
- offrir une bonne protection de l'appareil 100 qui s'y trouve contre les chocs, les chutes, les vibrations ou analogues, ou contre les intempéries, tel que pluie, neige..., et contre les salissures, tel que poussières, boue....
- être facile à transporter par l'utilisateur, i.e. le personnel soignant.
- être aisée à utiliser.
- ne pas obliger l'utilisateur à extraire l'appareil 100 pour pouvoir s'en servir.
- être solide et peu sensible aux déchirures ou autres.
- être conforme aux normes en vigueur, notamment les normes IEC 60601-1-12:2014 et NF EN 1789:2021

Enfin, Fig. 7 montre un appareil de délivrance de gaz, i.e. de NO, 100 pouvant être protégé par l'enceinte de protection 1 de Fig. 1, c'est-à-dire une enceinte de protection 1 selon l'invention.

Toutefois, cet exemple est non-limitatif et l'enceinte de protection 1 selon l'invention peut être utilisée pour protéger d'autres appareils de délivrance de gaz, en particulier de NO.

## Revendications

1. Enceinte (1) de protection pour un appareil de délivrance de gaz (100) médical à un patient, typiquement un appareil de délivrance de NO, comprenant une enveloppe protectrice (2) comprenant une façade avant (10) et un fond (50) et un compartiment interne (3) dimensionné et configuré pour loger un appareil de délivrance de gaz (100) médical, **caractérisée en ce que** :
- une première découpe frontale (6) est aménagée dans la façade avant (10) de l'enveloppe protectrice (2), et une vitre transparente en polymère (7) est agencée dans ladite première découpe (6),
- un premier élément amortisseur (4) est agencé sur le fond (50), à l'intérieur du compartiment interne (3), et
- un deuxième élément amortisseur (5) est agencé sur la façade avant (10) et fait saillie en éloignement par rapport à ladite façade avant (10) de l'enveloppe protectrice (2).

2. Enceinte selon la revendication 1, **caractérisée en ce que** le premier élément amortisseur (4) recouvre la majeure partie de la surface intérieure du fond (50), de préférence toute la surface intérieure du fond (50).

3. Enceinte selon la revendication 1, **caractérisée en ce qu'**un troisième élément amortisseur (8) est agencé sur la façade avant (10) et fait saillie en éloignement par rapport à ladite façade avant (10) de l'enveloppe protectrice (2).

4. Enceinte selon la revendication 3, **caractérisée en ce que** les deuxième (5) et troisième (8) éléments amortisseurs sont positionnés sur la façade avant (10), entre le fond (50) et la première découpe frontale (6) comprenant la vitre transparente (7).

5. Enceinte selon la revendication 3 ou 4, **caractérisée en ce qu'**une deuxième découpe frontale (9) est aménagée dans la façade avant (10) de l'enveloppe protectrice (2), ladite deuxième découpe frontale (9) étant située entre les deuxième et troisième éléments amortisseurs (5, 8).

6. Enceinte selon l'une quelconque des revendications 4 à 5, **caractérisée en ce que** le premier, deuxième et/ou le troisième élément amortisseur (4, 5, 8) comprennent une mousse amortissante.

7. Enceinte selon la revendication 1, **caractérisée en ce que** l'enveloppe protectrice (2) comprend la façade avant (10), une face arrière (20), un côté droit (30), un côté gauche (40) et le fond (50), reliés les uns aux autres, et un toit (60) agencé pivotant (XX) par rapport à la face avant (10).

8. Enceinte selon la revendication 7, **caractérisée en ce que** tout ou partie des quatre faces (10-40) de l'enveloppe est percée ou comprend une rangée de trous ou orifices (11) en communication fluidique avec le compartiment interne (3).

9. Enceinte selon la revendication 1, **caractérisée en ce que** l'enveloppe protectrice (2) est formée d'une toile comprenant des fibres textiles synthétiques, de préférence des fibres en polyester.

10. Enceinte selon la revendication 9, **caractérisée en ce que** les fibres textiles synthétiques sont enduites d'une substance protectrice, de préférence du polychlorure de vinyle (PVC).

11. Ensemble de ventilation (1, 100) comprenant un appareil de délivrance de gaz médical, typiquement un appareil de délivrance de NO (100), logé dans l'enveloppe protectrice (2) d'une enceinte (1) de protection selon l'une des revendications précédentes.

12. Utilisation d'une enceinte (1) de protection selon l'une des revendications 1 à 10, pour protéger un appareil de délivrance de gaz médical, typiquement un appareil de délivrance de NO (100).
